# NEW EUROPEAN PATENT SPECIFICATION

(11) **EP 1 446 063 B2**
(45) Date of publication and mention of the opposition decision: **04.12.2013**
(45) Mention of the grant of the patent: 14.12.2005
(21) Application number: 02793329.0
(22) Date of filing: 20.11.2002
(51) Int. Cl.: A61B 18/20

(54) **PULSED-LIGHT ELECTRIC MEDICAL APPLIANCE FOR SKIN TREATMENT**
PULSIERENDES LICHT ABSTRAHLENDER ELEKTROMEDINIZINISCHER APPLIKATOR ZUR HAUTBEHANDLUNG
DISPOSITIF MEDICAL ELECTRIQUE A LUMIERE PULSEE DESTINE AU TRAITEMENT DE LA PEAU

(30) Priority: 21.11.2001 IT BO20010706
(43) Date of publication of application: 18.08.2004
(73) Proprietor: GP Investimenti S.r.l., Montespertoli (FI) (IT)
(72) Inventor: NALDONI, Moreno, I-50018 Scandicci (IT)
(74) Representative: Bergadano, Mirko
(86) International application number: PCT/IT2002/000737
(87) International publication number: WO 2003/043514

(56) References cited:
- WO-A-90/12545
- WO-A-02/078786
- GB-A- 545 311
- US-A- 4 945 279
- US-B1- 6 187 001
- US-B1- 6 215 254
- US-B1- 6 280 438
- 'Spa touch TM Photoepilation System of Radiancy' MANUEL D'UTILISATION VERSION6, May 2000,

## Description

### TECHNICAL FIELD

The present invention relates to a pulsed-light electric medical appliance for skin treatment, and in particular for removing unwanted hair, reducing blotches, rejuvenating the skin, and vascular treatment,

### BACKGROUND ART

As is known, appliances of the above type provide for directing onto the skin area for treatment high-energy pulsed light in the ultraviolet to intermediate infrared wavelength range, and comprise a machine body housing the electric and electronic devices for producing the necessary light-generating energy; pulsed-light control electronics; and a handset outside the machine body, and which is applied to the skin and houses the light source defined by a gas or incandescent lamp. The handset is preferably fitted with one or more filters, made of glass or other suitable material, for cutting off given undesired wavelengths.

"Such an appliance is described, for instance in US-B1-6 280 438 (ECKHOUSE SHIMON ET AL). Indeed, such document describes an electric medical appliance for skin treatment, of the type comprising a handset having a lamp for generating, onto an area of a patient's skin high-energy pulsed light in an ultraviolet to intermediate infrared wavelength range, an electric start circuit for starting said lamp, and a filter defined by a sheet of glass and for cutting off light of given wavelengths." "Manuel d'utilisation" Spa Touch, dated May 2000, is a document upon which the preamble of claim 1 is based and which describes a photoepilation system. In particular, such a document discloses a pulsed-light electric medical appliance for skin treatment, comprising a handset housing a lamp for generating, onto an area of a patient's skin, high-energy pulsed light in an ultraviolet to intermediate infrared wavelength range, and an electric start circuit for starting the lamp.

Appliances of the above type have several drawbacks.

In particular, the lamp produces heat which may exceed 400°C, and which must be removed to prevent premature wear of certain parts of the handset, and to safeguard the patient against burns caused by contact with such parts. Moreover, the light applied to the skin penetrates the tissue and superheats the hair bulb, but also burns the hair on the outside of the skin, thus resulting in smoke which must be disposed of. Conventional appliances feature complex lamp cooling systems (Peltier cells, water, etc.) and equally complex skin cooling systems (Peltier cell rings or plates, or water); and handset-skin interface gel must also be used. As a result, the handsets are heavy, unwieldy, and complex to make. If no cooling systems are provided, the handsets may be fairly straightforward and easy to manage, but only at the expense of forgoing high power and separate optical filters, which, operating in contact with the skin and directly facing the power lamp, are subject to severe heating and a frequent cause of burns.

Moreover, fast-wear component parts are difficult to change, by either being housed inside the machine body, or forming part of a complex, high-cost assembly.

Some appliances feature a fast-change lamp, which may have a filter coating, but do not allow for using separate optical filters, and have a low power level.

### DISCLOSURE OF THE INVENTION

It is an object of the present invention to provide an electric medical appliance for skin treatment, designed to eliminate the aforementioned drawbacks, and which generates high power levels without forgoing the use of separate optical filters - extremely useful for adapting treatment to the patient's skin type. At the same time, the appliance according to the present invention features an extremely straightforward, low-cost air cooling system, and an innovative, compact, easy-change box assembly housing all the component parts subject to wear.

More specifically, the handset of the appliance according to the present invention comprises a grip; a head portion; and a box which is fitted and removed easily to and from the head portion, and houses the optical filter and electronic components requiring frequent replacement.

The box comprises a filter seat at such a distance from the skin as to prevent contact between the skin and filter; and a number of openings located at a rim of the box, and for cooling the inside of the box by outside air flowing through the gap between the optical filter and lamp.

According to the present invention, there is provided an electric medical appliance for skin treatment, according to Claim 1.

In addition to the lamp and relative start circuit, the box body also houses one or more superimposed filters, each defined by a sheet of glass and for cutting off light of given wavelengths. The present invention is characterized in that the box body housed inside said handset comprises an application window which is placed on the skin; and a support for said filters, which is located slightly higher than said window and formed in the walls of the box body.

### BRIEF DESCRIPTION OF THE DRAWINGS

A non-limiting embodiment of the present invention will be described by way of example with reference to the accompanying drawings, in which:
Figure 1 shows a view in perspective of an electric medical appliance for skin treatment, in accordance with the teachings of the present invention;
Figure 2 shows a larger-scale view in perspective of part of a handset of the Figure 1 appliance;
Figure 3 shows a longitudinal scale section of the Figure 1 appliance handset;
Figure 4 shows a cross section of a head portion of the Figure 3 handset;
Figure 5 shows an exploded view in perspective of the Figure 3 handset.

### BEST MODE FOR CARRYING OUT THE INVENTION

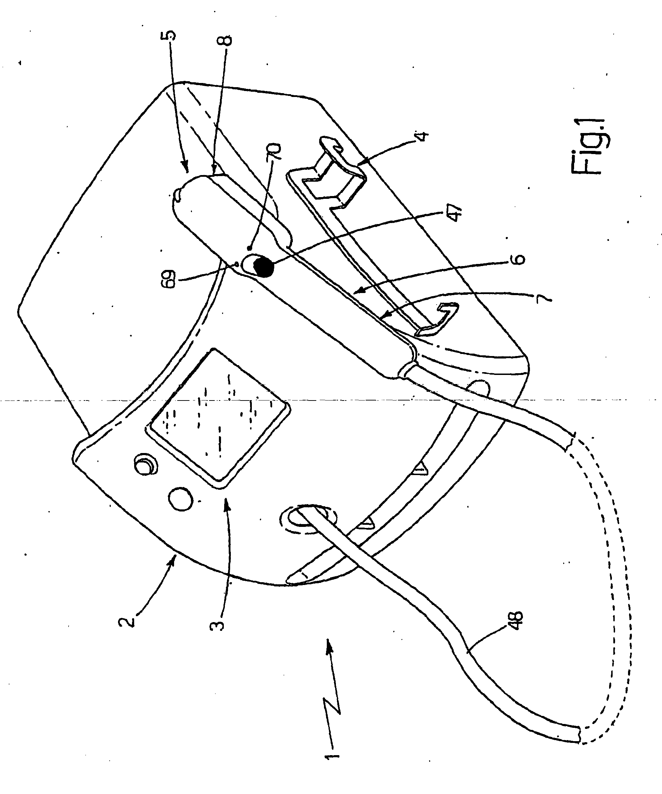

Number 1 in Figure 1 indicates as a whole a pulsed-light electric medical appliance for skin treatment, which may be used for removing unwanted hair, reducing blotches, rejuvenating the skin, vascular treatment, and other commonly practised applications, and which, as is known, provides for directing, onto the skin area for treatment, high-energy pulsed light in the ultraviolet to intermediate infrared wavelength range.

With reference to Figure 1, appliance 1 comprises a machine body 2 in which are housed an air intake pump; an electric power block; a block of discharge capacitors and relative control circuits; and a microprocessor block for controlling appliance 1 as a whole. All the above components inside machine body 2 are known types and therefore not shown. Machine body 2 supports a known - in particular, touch screen - control panel 3 having a number of touch buttons for controlling the above components, and a display showing treatment cycle stages and the values of various physical quantities involved; and a seat 4 for a handset 5 having a number of innovative characteristics as explained below.

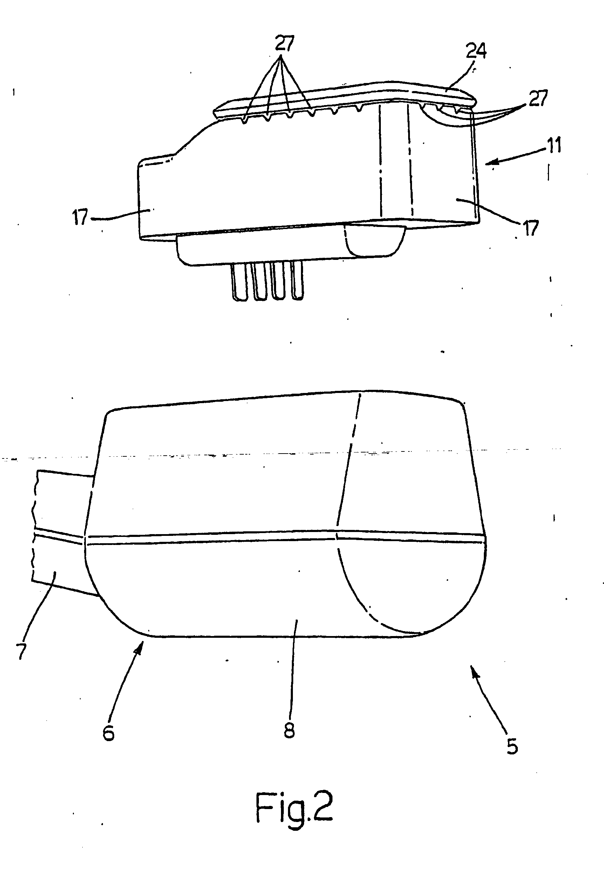

With reference to Figures 2 to 5, handset 5 comprises a shell 6 preferably made of plastic material in two parts 6a and 6b connected integrally by screws 6c. Shell 6 is defined by a grip 7 and a head portion 8, and handset 5 also comprises a box body 11 housed partly inside head portion 8 through an opening 12 in portion 8. Box body 11 houses a preferably U-shaped xenon lamp 13; an electric circuit 14 for starting lamp 13; and a filter 15 for cutting off light of wavelengths outside a given range, lamp 13 being a source of light in the ultraviolet to intermediate infrared wavelength range.

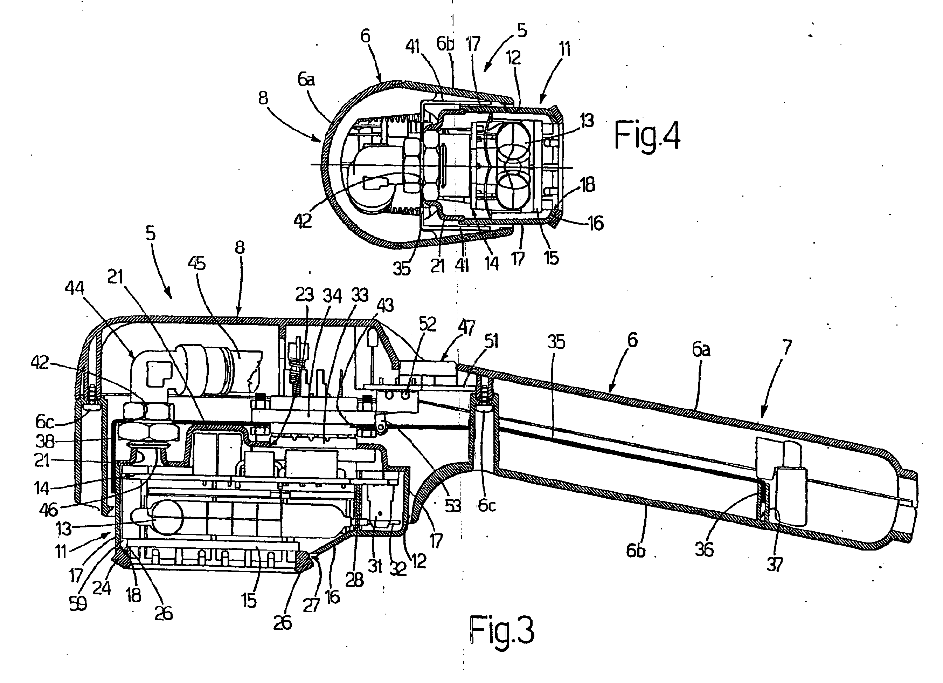

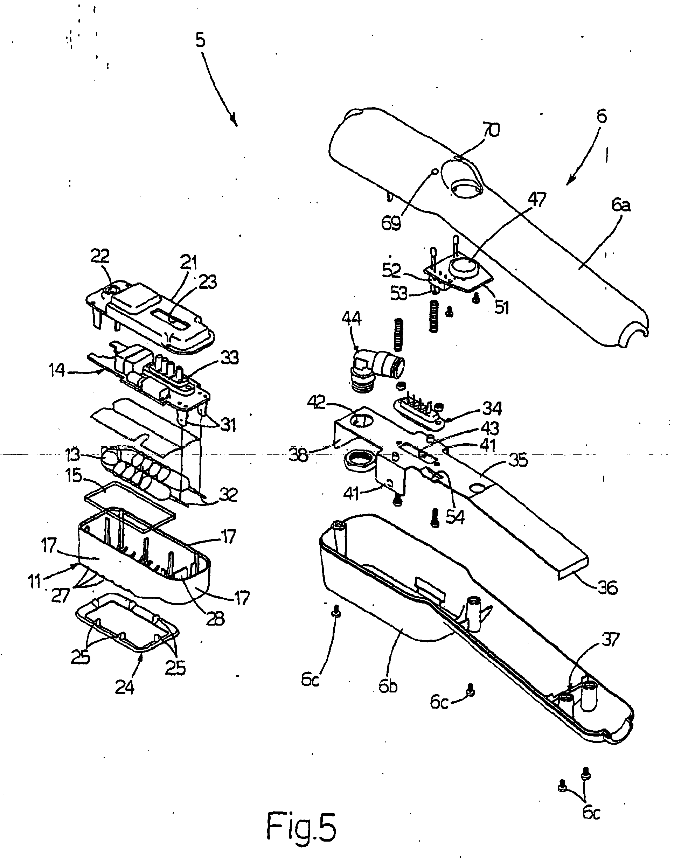

With reference to Figures 3 to 5, box body 11 comprises a base wall 16 and four lateral walls 17; a rectangular window 18 is formed in base wall 16; and box body 11 is closed at the top by a cover 21 in which are formed a through hole 22 and a through slot 23. At window 18, box body 11 is fitted inside with filter 15, which is defined by a sheet of glass fixed to a support 59 formed in lateral walls 17. A rim 24, fitted to window 18, has teeth 25 (Figure 5) which click inside appropriate seats 26 (Figure 3) formed along the edge of window 18; rim 24 is of such a thickness that its bottom edge is outside box body 11; and a number of air inlets 27 are formed along the edge of window 18 to allow the inside of box body 11 to communicate with the outside.

With reference to Figures 3 to 5, a wall 28 for supporting lamp 13 extends from base wall 16 inside box body 11. Start circuit 14 is installed between lamp 13 and cover 21, and is defined by a printed circuit having electric terminals 31 soldered to the electric terminals 32 of lamp 13, and an electric plug 33 for connection, through slot 23, to an electric socket 34 in head portion 8 of handset 5.

With reference to Figures 3 to 5, handset 5 is fitted inside with a bracket defined by a metal plate 35, a first axial end 36 of which is bent and engages a seat 37 in grip 7, and a second axial end 38 of which is bent and, together with two bent lateral tabs 41, encloses box body 11 inside head portion 8 of handset 5. That is, box body 11 is supported by end 38 and tabs 41 of plate 35, so that, together with plate 35 being elastically deformable, box body 11 can be positioned with a certain amount of slack inside head portion 8 of handset 5.

With reference to Figures 3 to 5, the part of plate 35 housed in head portion 8 comprises a through hole 42 coaxial with hole 22; and a slot 43 coaxial with slot 23. More specifically, at hole 42 is fixed a first end of a pneumatic fitting 44, a second end of which supports a tube 45 (shown partly for simplicity) connected pneumatically to the pump housed in machine body 2. By means of a seal 46 (Figure 3), the first end of fitting 44 is connected to hole 22, which has a raised edge; and socket 34 is fixed to plate 35 at slot 43.

With reference to Figures 3 and 5, head portion 8 of handset 5 comprises an electric pushbutton 47 operated, obviously, from the outside to electrically supply start circuit 14. More specifically, electric cables (not shown) extend from said electric power, discharge capacitor, and microprocessor blocks housed in machine body 2, and, outside machine body 2, are enclosed, together with tube 45, inside a sheath 48 (Figure 1), one end of which is fitted to the axial end of grip 7 so that tube 45 is connected to fitting 44, the electric cables from the microprocessor block are connected to corresponding contacts on socket 34, and the electric cables from the power and discharge capacitor blocks are connected to corresponding contacts on socket 34 by means of pushbutton 47.

With reference to Figures 3 and 5, head portion 8 is fitted inside with a small electric circuit 51 which on one side (Figure 1) supports pushbutton 47 and two LED's - a green LED 69 indicating the machine is ready, and a red LED 70 indicating the machine is charging - and on the opposite side supports a microswitch 52 electrically in series with pushbutton 47. Microswitch 52 has a pin 53 which is movable between a withdrawn position making the circuit electrically supplying circuit 14, obviously when pushbutton 47 is pressed, and an extracted position in which it breaks the supply circuit even when pushbutton 47 is pressed. Pin 53, or rather its outer axial end, cooperates with a recess 54 (Figure 5) formed in plate 35. In actual use, when the bottom edge of rim 24 is placed on the patient's skin, even with pushbutton 47 pressed, circuit 14 is not powered; whereas, when rim 24 is pressed with a certain amount of force on the skin, box body 11 withdraws inside head portion 8 and against plate 35 sufficiently to withdraw pin 53 by means of plate 35 and so power circuit 14 with pushbutton 47 pressed.

In actual use, after turning on a main switch on control panel 3, rim 24 is placed on the patient's skin, is then pressed with a certain amount of force on the skin, as described above, and pushbutton 47 is pressed to supply start circuit 14 and commence treatment. This system prevents light flashing in the air, which may cause severe eye injury to anyone in the vicinity of appliance 1. The air flowing through air inlets 27 towards the pump is first channeled into box body 11, flows over the inner face of filter 15 and lamp 13, and is channeled out of body 11 through fitting 44, so as to effectively remove the hot air produced by the powerful light pulses, and, by cooling lamp 13 and optical filter 15, cool the inside of body 11 to prevent overheating of the components inside, and remove any smoke produced during treatment. The microprocessor control electronics inside machine body 2 provide for maintaining airflow for a given software-set time period, even with the machine off, provided it is connected to the electricity mains. This feature, not to be found in any similar currently marketed appliances, provides for more effective cooling of lamp 13, optical filter 15, and the other components in box body 11, by also continuing cooling during downtime between treatments and whenever appliance 1 is not in use.

The advantages of the present invention will be clear from the foregoing description.

In particular, an appliance is provided comprising, in the handset, a box body housing all the components normally subject to wear and so requiring frequent replacement. Moreover, the box body is easy to assemble and disassemble to and from the handset, by being connected to it simply by means of a click-on fastener and an electric connector, and has a cooling system which not only prevents overheating of said components but also keeps the optical filter clean. Another innovative feature is the given distance maintained between the filter and skin, thus safeguarding the skin against burns, and, together with the cooling airflow described above, enabling other lamp and skin cooling systems to be dispensed with. Finally, the handset is provided with a safety system, as described above, which only enables treatment to commence when the handset is pressed with a certain amount of force on the skin.

## Claims

1. A pulsed-light electric medical appliance for skin treatment, comprising a handset (5) housing a lamp (13) for generating, onto an area of a patient's skin, high-energy pulsed light in an ultraviolet to intermediate infrared wavelength range, and an electric start circuit (14) for starting said lamp (13);
the appliance comprising, inside the handset (5),
a box body (11) housing said lamp (13); and whereby the handset (5) contains means (38, 41) for supporting and enabling withdrawal of said box body (11) from the head portion (8) of said handset (5) in one piece;
the box body (11) being capable of being pulled out of and pressed into said handset (5) in one piece;
said appliance being **characterised in that** the box body (11) further houses the electric start circuit (14) relative to said lamp (13), and an optical filter (15).

2. An appliance as claimed in Claim 1, **characterized in that** said optical filter (15) is located on a support (59) formed in walls (17) of said box body (11), so as to be located an appropriate distance from, and not contact, the skin during treatment.

3. An appliance as claimed in Claim 2, **characterized in that** said handset (5) comprises means (44, 45) for drawing air into said box body (11); the airflow being so channeled as to flow along the gap between the lamp (13) and the optical filter (15).

4. An appliance as claimed in Claim 3, **characterized by** employing a box body (11) containing a U-shaped lamp (13).

5. An appliance as claimed in Claim 4, **characterized in that** a number of air Inlets (27) are formed along the edge of a window (18) formed in said box body (11) to channel the air entering said box body (11) inwards.

6. An appliance as claimed in Claim 4, **characterized in that** said box body (11) is fitted inside, at a window (18) formed in said box body (11), with one or more filters (15), each defined by a sheet of glass, for selecting and adapting the light wavelength to the patient's skin by simply replacing the entire box body (11) fitted beforehand with a suitable filter (15).

7. An appliance as claimed in Claim 5, **characterized in that** said filter (15) and said lamp (13) continue to be cooled by said airflow for a given time, even after the treatment session, provided the appliance is connected to the electricity mains.

8. An appliance as claimed in any one of the foregoing Claims, **characterized in that** the handset (5) is fitted inside with a bracket defined by an elastically deformable metal plate (35); bent tabs (38, 41) extending from said plate (35) to enclose and support said box body (11); and said box body (11), when pressed onto the skin, withdrawing inside said handset (5),

9. An appliance as claimed in Claim 7, **characterized by** comprising a pushbutton (47) for controlling electric supply to said electric start circuit (14); and a microswitch (52) connected in series to said pushbutton (47) and for only permitting electric supply to said electric start circuit (14) upon withdrawal of said box body (11) inside said handset (5); said microswitch (52) having a pin (53) cooperating with said plate (35) to follow deformation of the plate and so make or break an electric contact in said microswitch (52).

## Patentansprüche

1. Pulsierendes Licht abstrahlender elektromedizinischer Gerät zur Hautbehandlung, umfassend ein Handteil (5), das eine Lampe (13) aufnimmt, um auf einem Bereich einer Haut eines Patienten hochenergetisches gepulstes bzw. pulsierendes Licht in einem Wellenlängenbereich von ultraviolett bis mittlerem Infrarot zu generieren bzw. zu erzeugen, und eine elektrische Startschaltung (14), um die Lampe (13) zu starten; wobei das Gerät im Inneren des Handteils (5) umfaßt
einen Kasten- bzw. Schachtelkörper (11), der die Lampe (13) aufnimmt; und wodurch das Handteil (5) Mittel (38, 41) zum Unterstützen und Ermöglichen einer Entnahme des Schachtelkörpers (11) von dem Kopfabschnitt (8) des Handteils (5) in einem Stück umfaßt;
wobei der Kasten- bzw. Schachtelkörper (11) in einem Stück einfach aus dem Handteil (5) herausgezogen und in dieses hinein gepreßt wird;
wobei das Gerät **dadurch gekennzeichnet ist, dass** der Kasten- bzw. Schachtelkörper (11) ferner die elektrische Startschaltung (14) relativ zu der Lampe (13) und ein optischer Filter (15) aufnimmt.

2. Gerät nach Anspruch 1, **dadurch gekennzeichnet, daß** der optische Filter (15) auf bzw. an einem Support bzw. Träger (59) angeordnet ist, der in Wänden (17) des Schachtelkörpers (11) ausgebildet ist, um in einem geeigneten Abstand von und nicht in Kontakt mit der Haut während einer Behandlung angeordnet zu sein.

3. Gerät nach Anspruch 2, **dadurch gekennzeichnet, daß** das Handteil (5) Mittel (44, 45) umfaßt, um Luft in den Schachtelkörper (11) zu ziehen; wobei der Luftstrom so kanalisiert ist, um entlang des Spalts zwischen der Lampe (13) und dem optischen Filter (15) zu fließen.

4. Gerät nach Anspruch 3, **gekennzeichnet durch** Verwenden eines Schachtelkörpers (11), der eine U-förmige Lampe (13) enthält.

5. Gerät nach Anspruch 4, **dadurch gekennzeichnet, daß** eine Anzahl der Lufteinlässe (27) entlang der Kante bzw. des Rands eines Fensters (18) ausgebildet ist, das in dem Schachtelkörper (11) ausgebildet ist, um die Luft, die in den Schachtelkörper (11) eintritt, nach innen zu kanalisieren.

6. Gerät nach Anspruch 4, **dadurch gekennzeichnet, daß** der Schachtelkörper (11) im Inneren, bei einem Fenster (18), das in dem Schachtelkörper (11) ausgebildet ist, mit einem oder mehreren Filter(n) (15) eingepaßt ist, welche(s) jeweils durch ein Glasblatt definiert ist bzw. sind, um die Lichtwellenlänge auf die Haut des Patienten durch ein einfaches Ersetzen des gesamten Schachtelkörpers (11) auszuwählen und zu adaptieren, der zuvor mit einem geeigneten Filter (15) versehen wurde.

7. Gerät nach Anspruch 5, **dadurch gekennzeichnet, daß** der Filter (15) und die Lampe (13) fortgesetzt durch den Luftstrom für einen gegebenen Zeitraum selbst nach einer Behandlungssitzung gekühlt sind, unter der Voraussetzung, daß das Gerät mit der Elektrizitäts-Netzversorgung verbunden ist.

8. Gerät nach einem der vorhergehenden Ansprüche, **dadurch gekennzeichnet, daß** das Handteil (5) im Inneren mit einer Klammer bzw. einem Träger versehen ist, die (der) durch eine elastisch deformierbare Metallplatte (35) definiert ist; wobei sich gebogene Fortsätze (38, 41) von der Platte (35) erstrecken, um den Schachtelkörper (11) zu umschließen und abzustützen; und wobei sich der Schachtelkörper (11), wenn er auf die Haut gepreßt wird, in das Innere des Handteils (5) zurückzieht.

9. Gerät nach Anspruch 7, **gekennzeichnet durch** ein Umfassen eines Druckknopfs (47), um eine elektrische Zufuhr zu der elektrischen Startschaltung (14) zu steuern bzw. zu regeln, und einen Mikroschalter (52), der in Serie mit dem Druckknopf (47) verbunden ist und eine elektrische Zufuhr bzw. Versorgung zu der elektrischen Startschaltung (14) nur nach Entfernen des Schachtelkörpers (11) in das Innere des Handteils (5) ermöglicht; wobei der Mikroschalter (52) einen Stift bzw. Zapfen (53) aufweist, der mit der Platte (35) kooperiert, um einer Deformation der Platte zu folgen und so einen elektrischen Kontakt in dem Mikroschalter (52) auszubilden oder zu unterbrechen.

## Revendications

1. Un système médical électrique à lumière pulsée pour traitement de la peau, comprenant un combiné (5) logeant une lampe (13) pour produire une lumière pulsée à haute énergie sur une portion de la peau d'un patient, dont la gamme de longueurs d'onde s'étend de l'ultraviolet à l'infrarouge intermédiaire, et un circuit de démarrage électrique (14) pour démarrer ladite lampe (13) ;
le système comprenant, à l'intérieur du combiné (5),
un corps ou carrossage en caisson (11) logeant ladite lampe (13), et dans lequel le combiné (5) contient un dispositif (38, 41) pour maintenir en place ledit corps ou carrossage en caisson (11) et permettre le retrait dudit corps ou carrossage en caisson (11) de la portion frontale (8) dudit combiné (5) en une seule pièce,
le corps ou carrossage en caisson (11) étant simplement retiré du ou pressé dans le combiné (5) en une seule pièce ;
ledit système étant **caractérisé en ce que** le corps ou carrossage en caisson (11) loge en outre le circuit de démarrage électrique (14) relatif à cette lampe (3), et un filtre optique (15).

2. Un système médical selon la revendication 1, **caractérisé en ce que** ledit filtre optique (15) est disposé sur un support (59) formé dans les parois (17) dudit carrossage ou corps en caisson (11), afin de le situer à une distance appropriée de la peau pendant le traitement, et non pas à son contact.

3. Un système médical selon la revendication 2, **caractérisé en ce que** ledit combiné (5) comprend un dispositif (44, 45) pour aspirer de l'air dans ledit carrossage ou corps en caisson (11) ; l'écoulement d'air étant canalisé pour s'écouler le long de l'espace présent entre la lampe (13) et le filtre optique (15).

4. Un système médical selon la revendication 3, **caractérisé en ce qu'**il utilise un corps ou carrossage en caisson (11) contenant une lampe en U (13).

5. Un système médical selon la revendication 4, **caractérisé en ce qu'**un nombre d'admissions d'air (27) sont formées le long de l'arête d'une fenêtre (18) formée dans ledit corps ou carrossage en caisson (11) pour canaliser l'air pénétrant dans ledit carrossage ou corps en caisson (11) vers l'intérieur.

6. Un système médical selon la revendication 4, **caractérisé en ce qu'**un ou plusieurs filtres (15) sont montés à l'intérieur dudit carrossage ou corps en caisson (11), au niveau d'une fenêtre (18) formée dans ledit carrossage en caisson (11), chacun étant défini par une plaque de verre, pour sélectionner la longueur d'onde et l'adapter à la peau du patient en remplaçant simplement le carrossage ou corps en caisson (11) en entier, auparavant équipé avec un filtre approprié (15).

7. Un système médical selon la revendication 5, **caractérisé en ce que** ledit filtre (15) et ladite lampe (13) continuent à être refroidis par ledit écoulement d'air pendant une période donnée, même après la session de traitement, à condition que le système médical soit couplé avec une source d'électricité.

8. Un système médical selon l'une quelconque des revendications précédentes, **caractérisé en ce qu'**un support défini par une plaque de métal élastiquement déformable (35) est monté à l'intérieur du combiné (5) ; des onglets pliés (38, 41) s'étendant depuis ladite plaque (35) pour ceindre et supporter ledit corps ou carrossage en caisson (11) et ledit carrossage en caisson (11) se retirant à l'intérieur dudit combiné (5) lorsqu'il est pressé contre la peau.

9. Un système médical selon la revendication 7, **caractérisé en ce qu'**il comprend un bouton-poussoir (47) pour commander l'alimentation d'électricité audit circuit de démarrage électrique (14) ; et un microrupteur (52) couplé en série avec ledit bouton-poussoir (47) pour permettre l'alimentation d'électricité audit circuit de démarrage électrique (14) uniquement lorsque ledit carrossage en caisson (11) se retire à l'intérieur dudit combiné (5) ; ledit microrupteur (52) possédant une broche (53) coopérant avec ladite plaque (35) pour suivre la déformation de la plaque et ainsi établir ou interrompre un contact électrique dans ledit microrupteur (52).
